# EUROPEAN PATENT SPECIFICATION

(11) **EP 3 354 243 B1**
(45) Date of publication and mention of the grant of the patent: **07.10.2026**
(21) Application number: 18157768.5
(22) Date of filing: 20.11.2008
(51) Int. Cl.: A61F 13/0206, A61F 13/0203, A61F 13/05, A61M 1/00

(54) **WOUND DRESSING**
WUNDAUFLAGE
PANSEMENT DE PLAIE

(30) Priority: 21.11.2007 GB 0722820; 17.09.2008 GB 0817020
(43) Date of publication of application: 01.08.2018
(62) Document number(s) of the earlier application(s) in accordance with Art. 76 EPC: 08851879.0 / 2 214 611
(73) Proprietor: Smith & Nephew plc, Watford, Hertfordshire WD18 8YE (GB)
(72) Inventor: HARTWELL, Edward, Yerbury, Hull, HU3 2BN (GB)
(74) Representative: HGF

(56) References cited:
- WO-A1-2006/056294
- GB-A- 2 415 908
- US-A1- 2007 185 463
- US-A1- 2007 265 585

## Description

The present invention relates to an apparatus for dressing a wound .

The present invention relates to a wound dressing useable during topical negative pressure (TNP) therapy in which the wound dressing itself acts as a waste canister to collect and store wound exudate removed from a wound site.

There is much prior art available relating to the provision of apparatus and methods of use thereof for the application of topical negative pressure (TNP) therapy to wounds together with other therapeutic processes intended to enhance the effects of the TNP therapy. Examples of such prior art include those listed and briefly described below.

TNP therapy assists in the closure and healing of wounds by reducing tissue oedema; encouraging blood flow and granulation of tissue; removing excess exudates and may reduce bacterial load and thus, infection to the wound. Furthermore, TNP therapy permits less outside disturbance of the wound and promotes more rapid healing.

In International patent application, WO 2004/037334, apparatus, a wound dressing and a method for aspirating, irrigating and cleansing wounds are described. In very general terms, the application describes the treatment of a wound by the application of TNP therapy for aspirating the wound together with the further provision of additional fluid for irrigating and/or cleansing the wound, which fluid, comprising both wound exudates and irrigation fluid, is then drawn off by the aspiration means and circulated through means for separating the beneficial materials therein from deleterious materials. The materials which are beneficial to wound healing are recirculated through the wound dressing and those materials deleterious to wound healing are discarded to a waste collection bag or vessel.

In International patent application, WO 2005/04670, apparatus, a wound dressing and a method for cleansing a wound using aspiration, irrigation and cleansing wounds are described. Again, in very general terms, the invention described in this document utilises similar apparatus to that in WO 2004/037334 with regard to the aspiration, irrigation and cleansing of the wound, however, it further includes the important additional step of providing heating means to control the temperature of that beneficial material being returned to the wound site/dressing so that it is at an optimum temperature, for example, to have the most efficacious therapeutic effect on the wound. In International patent application, WO 2005/105180, apparatus and a method for the aspiration, irrigation and/or cleansing of wounds are described. Again, in very general terms, this document describes similar apparatus to the two previously mentioned documents hereinabove but with the additional step of providing means for the supply and application of physiologically active agents to the wound site/dressing to promote wound healing.

US2007/0265585 discloses a wound therapy device including a housing for covering at least a portion of a wound and for sealing to a body surface of a patient and that may also include a liquid-retention chamber for retaining liquid therein and a vacuum connection for coupling to a vacuum source.

However, the above described apparatus are generally only applicable to a patient when hospitalised as the apparatus used is complex, needing people having specialist knowledge in how to operate and maintain the apparatus, and also relatively heavy and bulky, not being adapted for easy mobility outside of a hospital environment by a patient, for example.

Some patients having relatively less severe wounds which do not require continuous hospitalisation, for example, but whom nevertheless would benefit from the prolonged application of TNP therapy, could be treated at home or at work subject to the availability of an easily portable and maintainable TNP therapy apparatus. To this end GB-A-2 307 180 describes a portable TNP therapy unit which may be carried by a patient and clipped to belt or harness. A negative pressure can thus be applied at a wound site.

During TNP therapy a portable or non-portable therapy unit generates a negative pressure at a wound site. As fluid, including air as well as wound exudate material is removed from the wound site this must be collected in some manner remote from the wound site. With prior known therapy units the collection and storage of wound exudate material is typically carried out by a waste canister connected to a pump unit of the therapy unit. The use of a canister, however, can result in the therapy unit apparatus itself being quite bulky and expensive to manufacture. Also replacing a canister or a bag in a canister in which wound exudate is collected can be a time consuming and relatively unhygienic process.

Prior known therapy units also tend to include a pump which is used to generate the negative pressure. Such pumps can be costly to manufacture and are relatively heavy.

WO 2007/030601 discloses a self-contained wound dressing with a micro pump. The pump for drawing wound fluid into a vacuum zone is included in a wound dressing itself. Nevertheless wound exudate from the dressing can only be removed via a complex series of steps. The exudate removal process is also prone to contamination since once an absorbent layer is fully saturated with wound exudate an access door must be opened in the wound dressing so that the absorbent layer and micro pump can be removed. It will be appreciated that such exudate removal and pump removal can be time consuming and can lead to cross contamination between users. A further problem is that the wound dressing is prone to over expansion and rupture.

It is an aim of the present invention to at least partly mitigate the above-mentioned problems.

The invention is as defined in the claims.

Certain embodiments of the present invention provide the advantage that a disposable wound dressing can be fixed over a wound site and can simultaneously be used to provide negative pressure at the wound site and collect and store wound exudate. Certain embodiments of the present invention provide the advantage that a separate therapy unit is not required to generate negative pressure at a wound site and collect and store any wound exudate. Rather a wound dressing can carry out both a pumping and wound exudate collecting process. The wound dressing may then be a one use item which can be disposed of subsequent to use. This reduces a risk of contamination.

Certain embodiments of the present invention provide the advantage that a wound dressing can be used to collect wound exudate generated during a negative pressure therapy process. A pump remote from the wound dressing can be connected to the wound dressing and reused whilst the wound dressing itself is used to collect wound exudate and may then be disposed of after use.

Embodiments of the present invention will now be described hereinafter, by way of example only, with reference to the accompanying drawings in which:
Figure 1 illustrates a wound dressing;
Figure 2 illustrates a top view of a wound dressing;
Figure 3 illustrates a portion of the wound dressing;
Figure 4 illustrates an exploded view of a wound dressing with a mounted pump; and
Figure 5 illustrates a view of a horizontal section through a wound dressing.

In the drawings like reference numerals refer to like parts.

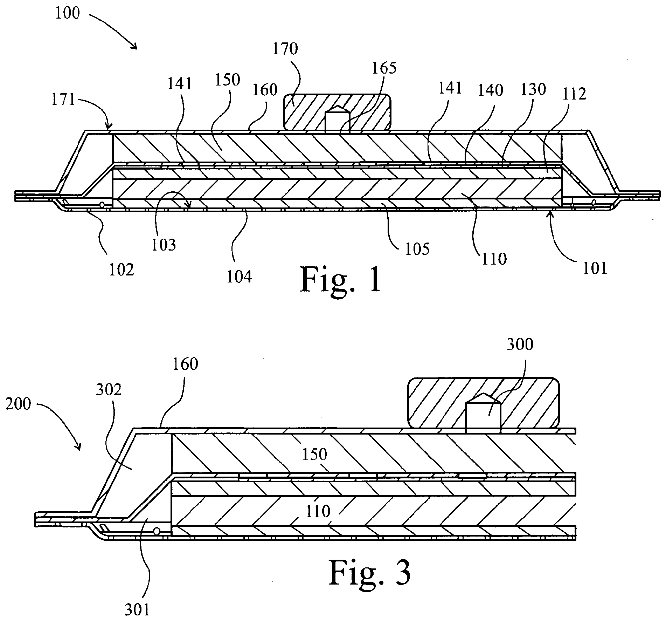

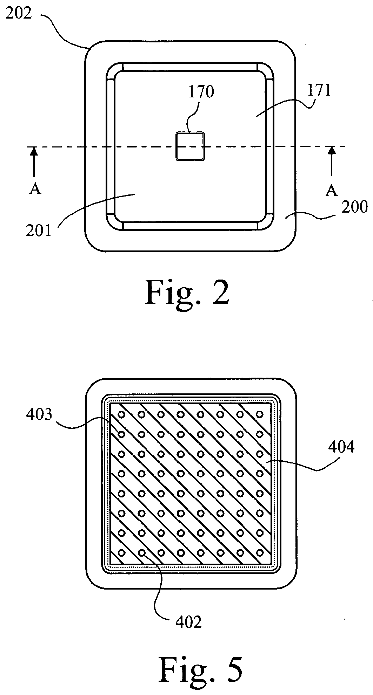

Figure 1 illustrates a cross section through a wound dressing 100 according to an embodiment of the present invention. A plan view from above of the wound dressing 100 is illustrated in Figure 2 with the line A-A indicating the location of the cross section shown in Figure 1. It will be understood that Figure 1 illustrates a generalised schematic view of an apparatus 100. It will be understood that embodiments of the present invention are generally applicable to use in topical negative pressure (TNP) systems. Briefly, negative pressure wound therapy assists in the closure and healing of many forms of "hard to heal" wounds by reducing tissue oedema; encouraging blood flow and granular tissue formation; removing excess exudate and reducing bacterial load (and thus infection risk). In addition, the therapy allows for less disturbance of a wound leading to more rapid healing.

The wound dressing 100 can be located over a wound site to be treated. The dressing 100 forms a sealed cavity over the wound site. Optionally wound packer can be used within a wound cavity below the dressing. Aptly the packer material can be a gauze or reticulated PU foam material.

It is envisaged that the negative pressure range for the apparatus embodying the present invention is between about -50mmHg and -200mmHg (note that these pressures are relative to normal ambient atmospheric pressure thus, -200mmHg would be about 560mmHg in practical terms). Aptly the pressure range may be between about -75mmHg and -150mmHg. Alternatively a pressure range of up to -75mmHg, up to -80mmHg or over -80mmHg can be used. Also aptly a pressure range of below -75mmHg could be used. Alternatively a pressure range of over -100mmHg could be used or over -150mmHg.

As illustrated in Figure 1 a lower surface 101 of the wound dressing 100 is provided by a perforated wound contact layer 102. The wound contact layer 102 can be a polyurethane layer or polyethylene layer or other flexible layer which is perforated, for example via a hot pin process or in some other way, or otherwise made permeable to liquid and gas. The wound contact layer has a lower surface 101 and an upper surface 103. The perforations 104 are through holes in the wound contact layer which enables fluid to flow through the layer. The wound contact layer helps prevent tissue ingrowth into the other material of the wound dressing. The perforations are small enough to meet this requirement but still allow fluid through. The wound contact layer also helps hold the whole wound dressing together and acts as a carrier for an optional lower and upper adhesive layer (not shown). A lower pressure sensitive adhesive is provided on the underside surface 101 of the wound dressing and/or an upper pressure sensitive adhesive layer is provided on the upper surface 103 of the wound contact layer. The pressure sensitive adhesive which may be a silicone or acrylic based adhesive or other such adhesives may be formed on both sides or optionally on a selected one or none of the sides of the wound contact layer. When a lower pressure sensitive adhesive layer is utilised this helps adhere the wound dressing to the skin around a wound site.

An layer 105 of porous material such as a foam layer or the like is located above the wound contact layer. This porous layer allows transmission of fluid including liquid and gas away from a wound site into upper layers of the wound dressing. The layer 105 also helps distribute pressure generated by a pump, mentioned in more detail below, so that a whole wound site sees an equalised negative pressure. Reticulated foam or a non-woven material which might be natural or synthetic can be used as the porous material of the porous layer 105.

A layer 110 of absorbent material is provided above the transmission layer 1 .

The absorbent material which may be a foam or non-woven natural or synthetic material and which may optionally include or be super-absorbent material forms a reservoir for fluid, particularly liquid, removed from the wound site. The material of the absorbent layer also prevents liquid collected in the wound dressing from flowing in a sloshing manner. The absorbent layer 130 also helps distribute fluid throughout the layer via a wicking action so that fluid is drawn from the wound site and stored throughout the absorbent layer. This prevents agglomeration in areas of the absorbent layer. Since in use the absorbent layer experiences negative pressures the material of the absorbent layer is chosen to absorb liquid under such circumstances. Superabsorber material is an example of such a material. Non superabsorber material can be utilised however even where significant negative pressures are envisaged. The material of the absorbent layer does not need to be hydrophilic. Aptly a material with connective open voids can be used. Aptly a material is used that can resist the compressive force of the negative pressure e.g. precompressed FT11M foam manufactured by Foam Techniques. Aptly the absorbent material is selected so that fluid is prevented from draining back out when the dressing is removed. It is to be noted that if a superabsorber is used such a material is able to expand against the compressive force of the negative pressure.

A further optional layer 112 of porous material such as a foam layer or the like is located above the absorbent layer 110. This porous layer allows transmission of fluid including liquid and gas away from a wound site into upper layers of the wound dressing. The layer 112 also helps distribute pressure generated by a pump, mentioned in more detail below, so that a whole wound site sees an equalised negative pressure. Reticulated foam or a non-woven material which might be natural or synthetic can be used as a porous material of the porous layer 112. The material may the same or different from the previously mentioned layer 105 of porous material.

A filter layer 130 is provided over the absorbent layer 110. The filter layer permits moisture vapour and gas but no liquid through. A suitable material for the filter material of the filter layer 130 is 0.2 micron Gore^{™} expanded PTFE from the MMT range. Larger pore sizes can also be used but these may require a secondary filter layer to ensure full bioburden containment. As wound fluid contains lipids it is preferable, though not essential, to use an oleophobic filter membrane for example 1.0 micron MMT-332 prior to 0.2 micron MMT-323. This prevents the lipids from blocking the hydrophobic filter.

It will be understood that other types of material could be used for the filter layer. More generally a microporous membrane can be used which is a thin, flat sheet of polymeric material, this contains billions of microscopic pores. Depending upon the membrane chosen these pores can range in size from 0.01 to more than 10 micrometers. Microporous membranes are available in both hydrophilic (water filtering) and hydrophobic (water repellent) forms. Aptly the wound dressing 100 according to certain embodiments of the present invention uses microporous hydrophobic membranes (MHMs). Numerous polymers may be employed to form MHMs. For example, PTFE, polypropylene, PVDF and acrylic copolymer. All of these optional polymers can be treated in order to obtain specific surface characteristics that can be both hydrophobic and oleophobic. As such these will repel liquids with low surface tensions such as multi-vitamin infusions, lipids, surfactants, oils and organic solvents.

MHMs block liquids whilst allowing air to flow through the membranes. They are also highly efficient air filters eliminating potentially infectious aerosols and particles. A single piece of MHM is well known as an option to replace mechanical valves or vents. Incorporation of MHMs can thus reduce product assembly costs improving profits and costs/benefit ratio to a patient.

The filter layer 130 thus enables gas to be exhausted upwards through the wound dressing. Liquid, particulates and pathogens however are contained in the dressing.

A gas impermeable sealing layer 140 extends across the width of the wound dressing. The sealing layer which may, for example, be a polyurethane film having a pressure sensitive adhesive on both sides is impermeable to gas and this layer thus operates to seal a wound cavity over which the wound dressing is placed. In this way an effective chamber is made beneath the sealing layer and between the sealing layer and a wound site where a negative pressure can be established. The sealing layer 140 is sealed to the filter layer 130. For example via adhesive or welding techniques. Gas leaving the dressing thus passes through the filter layer and sealing layer.

Aptly the material of the sealing layer can have a high moisture vapour permeability for example Elastollan (Trade name) SP9109 manufactured by BASF. A dotted pattern spread acrylic adhesive can optionally be used to help improve moisture vapour permeability. An advantage of using a high moisture vapour permeability material as the sealing layer 160 is that the fluid handling capacity of the dressing may be increased significantly by the action of moisture transpiring through the film and dispersing into the atmosphere. Advantageously, transpiration rates can be easily achieved of the order of 3000 grams/centimetre square/24 hours as a result of the high humidity achieved in the dressing and intimate contact of material achieved during use of the apparatus at a negative pressure of up to 250mmHg below atmospheric pressure.

As illustrated in Figure 1 a grid array of through holes 141 are provided in the sealing layer. These enable fluid including gas and liquid to pass through the sealing layer 140. Alternatively where a separate cover layer and sealing layer are used the sealing layer may extend around only a circumferential area of the underlying layers where it seals between an outer layer (mentioned below in more detail) and the filter layer. As a result any gas leaving the wound site must leave via the filter layer. Liquid is retained in layers below the filter layer.

A layer 150 of porous material such as a foam layer or the like is located above the sealing layer 140. This porous layer allows transmission of fluid including liquid and gas away from a wound site. The layer 150 also helps distribute pressure generated by a pump, mentioned in more detail below, so that a whole wound site sees an equalised negative pressure. Reticulated foam or a non-woven material which might be natural or synthetic can be used as the porous material of the layer 150. The material may be the same or different from the material of the underlying layers 105, 112.

A cover layer 160 covers the absorbent layer of the wound dressing 100. The cover layer which, for example, may be a polyurethane film acts as a bacterial barrier and helps hold in liquid to stop fouling. The cover layer also provides integrity for the dressing and is impermeable to moisture vapour and gas. The cover layer helps hold the wound dressing together thus providing structural integrity. An upper surface 171 also presents a bacteria free non soiling surface. As an alternative the material of the cover layer can have a high moisture vapour permeability, for example Elastollan (Trade name) SP9109 manufactured by BASF. A dotted pattern spread acrylic adhesive can optionally be used to help improve moisture vapour permeability. An advantage of using a high moisture vapour permeability material as the cover layer 160 is that the fluid handling capacity of the dressing may be increased significantly by the action of moisture transpiring through the film and dispersing into the atmosphere. Advantageously, transpiration rates can be easily achieved of the order of 3000 grams/centimetre square/24 hours as a result of the high humidity achieved in the dressing and intimate contact of material achieved during use of the apparatus at a negative pressure of up to 250mmHg below atmospheric pressure.

A single aperture 165 formed as a single hole or close arrangement of holes is formed in a central region of the upper cover layer. The aperture 165 is in fluid communication with an inlet to a pump 170 which is mounted on the upper surface 171 of the cover layer. In operation the pump 170 pumps fluid through the wound dressing from a wound site below the wound contact layer 102 upwards through the first transmission layer 105, absorbent layer 110, further transmission layer 112, filter layer 130, sealing layer 140, and further transmission layer 150.

Turning to Figure 2 which illustrates a wound dressing 100 in accordance with an embodiment of the present invention one can see the upper surface 171 of the cover layer 170 which extends radially outwardly away from a centre of the dressing into a border region 200 surrounding a central raised region 201 overlying the foam layers and layer 110 of absorber. Figure 2 also helps illustrate the location of the pump 170 on the cover layer. As indicated in Figure 2 the general shape of the wound dressing is a square having equal side lengths with rounded corner regions 202. It will be appreciated that wound dressings according to other embodiments of the present invention can be shaped differently such as rectangular, circular or elliptical dressings.

Figure 3 illustrates an expanded view of the border region 200 of the wound dressing 100 illustrated in Figures 1, 2 and 3. As seen, the cover layer 160 extends over the foam transmission layer 150 into an edge region. Here the cover layer is secured to the sealing layer 140 and the wound contact layer 102. Figure 3 also helps illustrate how the perforations 104 in the wound contact layer 102 extend around the foam layer 105 and absorbent layer 110. It will be noted that a space 301 is indicated in Figure 3 underneath the sealing layer 140 and above the wound contact layer 102 and ends of the transmission layers 105, 112 and absorbent layer 110. The space 301 is shown for illustrative reasons only and in practice the transmission layers and absorbent layers will be bevelled somewhat so as to reduce the space. A further space 302 is likewise illustrated in Figure 3 above the sealing layer and below the inner surface of the cover layer. Again this is included for illustration only and in practice these spaces will be avoided due to a nipping process in the method of manufacture. It will also be appreciated by those skilled in the art that when put in use the wound dressing will be subject to a negative pressure within a region defined by the inner surface of the cover layer. Such a negative pressure will tend to collapse any remaining spaces.

It will be understood that according to embodiments of the present invention the wound contact layer is optional. This layer is, if used, porous to water and faces an underlying wound site. A lower porous layer 105 such as a reticulated PU foam layer is used to distribute gas and fluid removal such that all areas of a wound are subjected to equal pressure. The sealing layer together with the filter layer forms a substantially liquid tight seal over the wound. Thus as the pump 170 pumps a negative pressure is generated below the sealing layer. This negative pressure is thus experienced at the target wound site. Fluid including air and wound exudate is drawn through the wound contact layer and reticulated foam layer 105. The wound exudate drawn through the lower layers of the wound dressing is dissipated and absorbed into the absorbent layer where it is collected and stored. Air and moisture vapour is drawn upwards through the wound dressing through the intermediate transmission layer 112 and through the filter layer and sealing layer. The filter layer and sealing layer are secured together so as to prevent upward movement through the wound dressing of anything other than moisture vapour and air. This air and moisture vapour is drawn upwards by the pump 170 into the fluid inlet 300. The pump exhausts the fluid as air and moisture vapour through a fluid exit (not shown).

It is to be noted that the upper transmission layer 150 and cover layer 160 are optional. Their use is helpful during multi orientation use when portions of the filter layer might otherwise become occluded. When a separate cover layer 160 is not utilised the sealing layer also performs the extra function of covering the dressing and will thus additionally act as a cover layer.

It will be appreciated by those skilled in the art that rather than have a cover layer overlying the filter layer the cover layer may itself be overlain by a filter layer. The cover layer may thus be the outermost layer of the wound dressing or the filter layer may be the outermost layer of the wound dressing. Further outer layers (not shown) may optionally be used so long as they are gas and water vapour permeable.

As still further options the dressing can contain anti-microbial e.g. nanocrystalline silver agents on the wound contact layer and/or silver sulphur diazine in the absorbent layer. These may be used separately or together. These respectively kill micro-organisms in the wound and micro-organisms in the absorption matrix. As a still further option other active components, for example, pain suppressants, such as ibuprofen, may be included. Also agents which enhance cell activity, such as growth factors or that inhibit enzymes, such as matrix metalloproteinase inhibitors, such as tissue inhibitors of metalloproteinase (TIMPS) or zinc chelators could be utilised. As a still further option odour trapping elements such as activated carbon, cyclodextrine, zealite or the like may be included in the absorbent layer or as a still further layer above the filter layer.

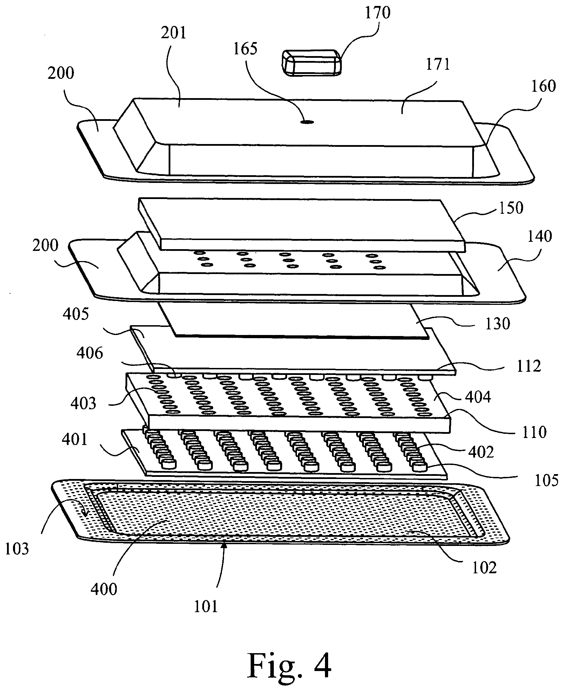

Figure 4 illustrates an exploded view of the wound dressing illustrated in Figures 1, 2 and 3. As illustrated in Figure 4 the lower-most layer of the wound dressing is a perforated wound contact layer 102. It will be appreciated that prior to use a still lower protective layer may be secured to the lower surface 101 of the wound contact layer. The protective paper (not shown) is removed immediately prior to application of the wound dressing over a wound site. During manufacture a central region 400 of the wound contact layer 102 is made slightly concave so as to provide a dished upper surface 103 for the wound contact layer.

A transmission layer 105 is duly located in the dished central region 400 of the wound contact layer. The foam layer includes a substantially rectangular base region 401 together with an array of upstanding columns 402. As illustrated in Figure 4 an array of 8 x 8 columns 402 may be used. It will be appreciated that other numbers of columns may be utilised. The columns 402 have a substantially circular cross section although it will be appreciated that column elements having different cross section shapes could be used. The column elements 402 and base section 402 are aptly integrally formed although these could be separately formed with the column elements being secured to the base section in some appropriate way such as via adhesive techniques.

The absorbent layer 110 is located above the transmission layer 105. The absorbent layer 110 is a layer of absorbent material and includes through holes 403 formed in a substantially rectangular block 404 of absorbent material. The through holes are set out in an 8 x 8 array to coincide with the upstanding columns 402 in the underlying transmission layer. It will be appreciated that the number and pattern of through bores 403 is selected to tally with the shape and number and arrangement of the columns.

The intermediate transmission layer 112 is a substantially rectangular base section 405 of porous material such as reticulated foam with an array of columns 406 extending downwardly from a lower surface of the base 405. The columns 406 coincide with locations of the through bores 403 in the absorbent layer. It will be appreciated that the columns 406 of the intermediate transmission layer 112 may be integrally formed with the base portion 405 of the transmission layer or may be secured in some fashion thereto. The height of the columns 402, 406 of the lower and intermediate transmission layers respectively is such that an upper contact surface of the columns 402 of the lower transmission layer and a lower contact surface of the columns 406 of the intermediate transmission layer contact when the wound dressing is put together.

These thus provide fluid transmission paths through the absorber layer so that fluid, including air and wound exudate and liquid, is drawn from the lower region upwardly through the absorbent layer when the pump 170 is operating.

A rectangular layer of filter material 130 is located above the upper surface of the base section 405 of the intermediate transmission layer. The filter layer blocks movement therethrough of liquid. The filter layer is aptly a 0.2 micron Gore^{™} expanded PTFE sheet.

A sealing layer 140 is located over the filter layer 130. The sealing layer has a border region and a generally concave central region 407. The underside of the sealing layer 140 is thus recessed. An array of apertures set out in a 5 x 5 grid array is made through the sealing layer 140. The sealing layer away from the apertures is gas and fluid tight. If a material having a high moisture vapour permeability is optionally used then the sealing layer will of course be permeable to moisture vapour. Fluid, including liquid and gas, can of course penetrate through the perforations. The filter layer 130 which is secured on the underside of the sealing layer, however, prevents liquid penetration through the apertures and to an extent prevents penetration of air through the apertures. Moisture vapour can penetrate through the apertures.

An upper transmission layer formed as a sheet of reticulated foam is located over the central region of the upper surface of the sealing layer 140. The upper transmission layer acts as a manifold and diffuser to help spread the negative pressure generated by the pump 170.

A cover layer 160 is located over the sealing layer and upper transmission layer 150. The cover layer has a border region 200 and a central raised region 201. The underside of the cover layer thus presents a central dished region to receive the upper transmission layer, raised central region of the sealing layer and the filter layer, intermediate transmission layer, absorbent layer and lower transmission layer. A central aperture 165 is made in the centre of the upper surface of the cover layer. The central aperture 165 is located to coincide with a fluid inlet 300 of the pump 170. Thus in use when a pump 170 is in use a negative pressure is generated under the cover 160. This negative pressure is distributed throughout the wound dressing and at a target wound site located under the wound contact layer. As the negative pressure is established and maintained wound exudate and air is drawn upwards away from the wound site through the wound dressing. Liquid and air is drawn upwards through the wound contact layer into the base of the lower transmission layer 105 and upwards through the connecting columns in the lower transmission layer and intermediate transmission layer. It will be appreciated of course that columns having a height sufficient to bridge the whole of the absorbent layer could be provided on either the upper surface of the lower transmission layer 105 or the lower surface of the intermediate transmission layer 112. Alternatively the apertures 403 in the absorbent layer may be filled with transmissive material such as foam cylinders when the wound dressing is manufactured. Any wound exudate being drawn upward through the wound dressing is dissipated outwardly from the absorbent material in the aperture regions of the absorbent layer. The liquid is thus collected and stored in the absorbent layer. Air and moisture vapour carries on upwards through the filter layer 130 and sealing layer 140 and is evacuated by the pump 170.

Figure 5 illustrates a horizontal cross section through the wound dressing illustrating an upper surface 404 of the absorbent layer including apertures 403. Each of the apertures 403 is filled with absorbent material such as columns 402 from the lower intermediate layer.

It will be appreciated that according to certain embodiments of the present invention fluid communication paths through which fluid can be transmitted from the lower transmission layer to the intermediate transmission layer can be made by pinching together peripheral regions of the lower and intermediate regions. Fluid transmission would thus proceed around the peripheral edges of the wound dressing. Such fluid paths may replace the fluid paths formed by the columns passing through apertures in the absorbent layer or may alternatively take the place of such passageways. This would maximise the quantity of absorber material in the layer 110 in the resultant wound dressing.

It is to be noted that according to certain other embodiments of the present invention a remote pump may be mounted to a border region of the wound dressing rather than onto the top surface. In such case tubes may be connected directly to the pump. Subsequent to a single use the wound dressing and pump may thus be discarded. As an option the tubes may be provided with a click fit connector or other easy fit connector which can be connected to corresponding mating connectors joined via corresponding tubes to a remote pump. In this way a remote pump may be reused whilst the wound dressing itself including connecting tubes and connectors is disposable after a single use.

It will be appreciated that alternatively the tubes could be provided by a single dual lumen tube. As a still further alternative the tubes may be provided by a single continuous looped tube, the tube then passing through pinch rollers for a peristaltic pump.

It will be understood that for embodiments of the present invention which include a pump mounted on the cover layer or on a peripheral border area of the dressing an integral power source and control circuitry can be included. Alternatively the power source can be external to the pump and remotely mounted. A remote power source and/or control circuitry improves the disposability of the dressing and permits battery recharge if spare batteries are used.

It is to be noted that in use the dressing may be used "up-side down", at an angle or vertical. References to upper and lower are thus used for explanation purposes only.

Where a separate cover layer and sealing layer are utilised such layers may be manufactured from the same or different materials.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

## Claims

1. Apparatus for dressing a wound and for use in the application of topical negative pressure therapy, comprising:
an absorbent layer (110) for absorbing wound exudate;
a liquid impermeable, gas permeable filter layer (130) over the absorbent layer;
a first liquid and gas permeable transmission layer (105) underlying the absorbent layer; wherein the first transmission layer is in fluid communication with the filter layer; and
a cover layer (160) comprising at least one orifice;
wherein the absorbent layer is in fluid communication with the filter layer; and
further comprising:
a perforated wound contact layer (102) under the first transmission layer and an adhesive layer on a lower surface and/or upper surface of the wound contact layer;
wherein the apparatus is configured to generate a negative pressure at the wound site in the range of between -50mmHg and -200mmHg.

2. The apparatus of claim 1, comprising an adhesive layer on a upper surface of the wound contact layer.

3. The apparatus of claim 1, comprising an adhesive layer on a lower surface and an upper surface of the wound contact layer.

4. The apparatus as claimed in any preceding claim further comprising:
a releasable protective layer on a lower surface of the wound contact layer.

5. The apparatus as claimed in any preceding claim, wherein the cover layer is over the filter layer or the filter layer is over the cover layer.

6. The apparatus as claimed in any preceding claim, wherein the absorbent layer is above the first transmission layer.

7. The apparatus as claimed in any preceding claim, wherein the absorbent layer is configured to distribute fluid throughout the layer via a wicking action so that fluid is drawn from the wound site and stored throughout the absorbent layer.

8. The apparatus of claim 7, wherein the absorbent layer is a synthetic material that includes or is superabsorbent material.

9. The apparatus as claimed in any preceding claim, wherein the material of the absorbent layer includes connective open voids.

10. The apparatus as claimed in any preceding claim, wherein the cover layer comprises a material having a high moisture vapour permeability.

11. The apparatus as claimed in any preceding claim, wherein the filter layer is over the absorbent layer and is configured to permit moisture vapour and gas but no liquid through.

12. The apparatus as claimed in any preceding claim wherein the apparatus includes an integrated pump to generate said negative pressure at the wound site.

13. The apparatus as claimed in any preceding claim, further comprising: a pump element on the cover layer, comprising an inlet in fluid communication with the orifice in the cover layer.

14. The apparatus as claimed in any of claims 1 to 11, further comprising: an outlet valve on the cover layer in fluid communication with the orifice in the cover layer and connectable to an extract conduit in fluid communication with a remote pump element.

## Patentansprüche

1. Vorrichtung zum Verbinden einer Wunde und zur Verwendung bei der Anwendung einer topischen Unterdrucktherapie, umfassend:
eine absorbierende Schicht (110) zum Absorbieren von Wundexsudat;
eine flüssigkeitsundurchlässige, gasdurchlässige Filterschicht (130) über der absorbierenden Schicht;
eine erste flüssigkeits- und gasdurchlässige Transmissionsschicht (105), die unter der absorbierenden Schicht liegt; wobei die erste Transmissionsschicht in Fluidverbindung mit der Filterschicht steht; und
eine Deckschicht (160), die mindestens eine Öffnung umfasst;
wobei die absorbierende Schicht in Fluidverbindung mit der Filterschicht steht; und
ferner umfassend:
eine perforierte Wundkontaktschicht (102) unter der ersten Transmissionsschicht und eine Klebeschicht auf einer unteren Oberfläche und/oder oberen Oberfläche der Wundkontaktschicht;
wobei die Vorrichtung dazu konfiguriert ist, einen Unterdruck an der Wundstelle in dem Bereich zwischen -50 mmHg und -200 mmHg zu erzeugen.

2. Vorrichtung nach Anspruch 1, umfassend eine Klebeschicht auf einer oberen Oberfläche der Wundkontaktschicht.

3. Vorrichtung nach Anspruch 1, umfassend eine Klebeschicht auf einer unteren Oberfläche und einer oberen Oberfläche der Wundkontaktschicht.

4. Vorrichtung nach einem vorhergehenden Anspruch, ferner umfassend: eine lösbare Schutzschicht auf einer unteren Oberfläche der Wundkontaktschicht.

5. Vorrichtung nach einem vorhergehenden Anspruch, wobei sich die Deckschicht über der Filterschicht befindet oder sich die Filterschicht über der Deckschicht befindet.

6. Vorrichtung nach einem vorhergehenden Anspruch, wobei sich die absorbierende Schicht über der ersten Transmissionsschicht befindet.

7. Vorrichtung nach einem vorhergehenden Anspruch, wobei die absorbierende Schicht dazu konfiguriert ist, Fluid über eine Dochtwirkung in der gesamten Schicht zu verteilen, so dass Fluid von der Wundstelle angesaugt und in der gesamten absorbierenden Schicht gespeichert wird.

8. Vorrichtung nach Anspruch 7, wobei die absorbierende Schicht ein synthetisches Material ist, das superabsorbierendes Material beinhaltet oder ist.

9. Vorrichtung nach einem vorhergehenden Anspruch, wobei das Material der absorbierenden Schicht offene verbunden Hohlräume beinhaltet.

10. Vorrichtung nach einem vorhergehenden Anspruch, wobei die Deckschicht ein Material umfasst, das eine hohen Feuchtigkeitsdampfdurchlässigkeit aufweist.

11. Vorrichtung nach einem vorhergehenden Anspruch, wobei sich die Filterschicht über der absorbierenden Schicht befindet und dazu konfiguriert ist, Feuchtigkeitsdampf und Gas, aber keine Flüssigkeit durchzulassen.

12. Vorrichtung nach einem vorhergehenden Anspruch, wobei die Vorrichtung eine integrierte Pumpe beinhaltet, um den Unterdruck an der Wundstelle zu erzeugen.

13. Vorrichtung nach einem vorhergehenden Anspruch, ferner umfassend: ein Pumpenelement auf der Deckschicht, das einen Einlass in Fluidverbindung mit der Öffnung in der Deckschicht umfasst.

14. Vorrichtung nach einem der Ansprüche 1 bis 11, ferner umfassend: ein Auslassventil an der Deckschicht in Fluidverbindung mit der Öffnung in der Deckschicht und verbindbar mit einer Extraktionsleitung in Fluidverbindung mit einem entfernten Pumpenelement.

## Revendications

1. Appareil permettant de panser une plaie et destiné à être utilisé dans l'application d'une thérapie topique à pression négative, comprenant :
une couche absorbante (110) destinée à absorber l'exsudat de la plaie ;
une couche filtrante imperméable aux liquides et perméable aux gaz (130) sur la couche absorbante ;
une première couche de transmission perméable aux liquides et aux gaz (105) sous-jacente à la couche absorbante ;
ladite première couche de transmission étant en communication fluidique avec la couche filtrante ; et
une couche de recouvrement (160) comprenant au moins un orifice ;
ladite couche absorbante étant en communication fluidique avec la couche filtrante ; et
comprenant en outre :
une couche de contact de plaie perforée (102) sous la première couche de transmission et une couche adhésive sur une surface inférieure et/ou une surface supérieure de la couche de contact de plaie ;
ledit appareil étant conçu pour générer une pression négative au niveau du site de la plaie comprise dans la plage entre -50 mm de Hg et -200 mm de Hg.

2. Appareil selon la revendication 1, comprenant une couche adhésive sur une surface supérieure de la couche de contact de plaie.

3. Appareil selon la revendication 1, comprenant une couche adhésive sur une surface inférieure et une surface supérieure de la couche de contact de plaie.

4. Appareil selon l'une quelconque des revendications précédentes, comprenant en outre :
une couche protectrice libérable sur une surface inférieure de la couche de contact de plaie.

5. Appareil selon l'une quelconque des revendications précédentes, ladite couche de recouvrement se trouvant au-dessus de la couche filtrante ou la couche filtrante se trouvant au-dessus de la couche de recouvrement.

6. Appareil selon l'une quelconque des revendications précédentes, ladite couche absorbante se trouvant au-dessus de la première couche de transmission.

7. Appareil selon l'une quelconque des revendications précédentes, ladite couche absorbante étant conçue pour distribuer un fluide à travers la couche par un effet de mèche de sorte que le fluide soit aspiré à partir du site de la plaie et stocké à travers la couche absorbante.

8. Appareil selon la revendication 7, ladite couche absorbante étant un matériau synthétique qui comprend ou est un matériau super-absorbant.

9. Appareil selon l'une quelconque des revendications précédentes, ledit matériau de la couche absorbante comprenant des vides ouverts connectifs.

10. Appareil selon l'une quelconque des revendications précédentes, ladite couche de recouvrement comprenant un matériau présentant une perméabilité élevée à la vapeur d'eau.

11. Appareil selon l'une quelconque des revendications précédentes, ladite couche filtrante se trouvant au-dessus de la couche absorbante et étant conçue pour laisser passer la vapeur d'eau et le gaz mais pas de liquide.

12. Appareil selon l'une quelconque des revendications précédentes, ledit appareil comprenant une pompe intégrée destinée à générer ladite pression négative au niveau du site de la plaie.

13. Appareil selon l'une quelconque des revendications précédentes, comprenant en outre : un élément de pompe sur la couche de recouvrement, comprenant une entrée en communication fluidique avec l'orifice dans la couche de recouvrement.

14. Appareil selon l'une quelconque des revendications 1 à 11, comprenant en outre : une soupape de sortie sur la couche de recouvrement en communication fluidique avec l'orifice dans la couche de recouvrement et pouvant être connectée à un conduit d'extraction en communication fluidique avec un élément de pompe distant.
